(19) **Europäisches Patentamt**

**European Patent Office**

**Office européen des brevets**

(11) **EP 0 832 646 A1**

(12) **EUROPEAN PATENT APPLICATION**
published in accordance with Art. 158(3) EPC

(43) Date of publication:
01.04.1998 Bulletin 1998/14

(51) Int. Cl.$^6$: **A61K 7/48**, A61K 7/00

(21) Application number: 97914571.1

(86) International application number:
PCT/JP97/01115

(22) Date of filing: 31.03.1997

(87) International publication number:
WO 97/37635 (16.10.1997 Gazette 1997/44)

(84) Designated Contracting States:
DE ES FR GB IT NL

(30) Priority: 04.04.1996 JP 108359/96

(71) Applicants:
• SHISEIDO COMPANY LIMITED
Tokyo 104-10 (JP)
• NIPPON SUISAN KAISHA, LTD.
Tokyo 100 (JP)

(72) Inventors:
• YOSHIDA, Yuzou,
c/o Shiseido Company Ltd.
Yokohama-shi, Kanagawa 223 (JP)
• KITAMURA, Kenji,
c/o Shiseido Company Ltd.
Yokohama-shi, Kanagawa 223 (JP)

• YOKOKAWA, Yoshihiro,
c/o Shiseido Company Ltd.
Yokohama-shi, Kanagawa 223 (JP)
• OTA, Masahiro,
c/o Shiseido Company Ltd.
Yokohama-shi, Kanagawa 223 (JP)
• DOSHIDA, Jyunko,
c/o Nippon Suisan Kaisha, Ltd.
Hachioji-shi, Tokyo 192 (JP)
• SHIMIZU, Nobuyoshi,
c/o Nippon Suisan Kaisha, Ltd.
Hachioji-shi, Tokyo 192 (JP)
• HATA, Kazuhiko,
c/o Nippon Suisan Kaisha, Ltd.
Hachioji-shi, Tokyo 192 (JP)

(74) Representative:
TER MEER STEINMEISTER & PARTNER GbR
Mauerkircherstrasse 45
81679 München (DE)

(54) **EXTERNAL PREPARATIONS FOR THE AMELIORATION AND PREVENTION OF CHAPPED SKIN**

(57) An external preparations for the amelioration and prevention of chapped skin contains as an effective ingredient an extract from a seaweed of genus Ascophyllum. The external preparation has a superior chapped skin amelioration action and superior effects on amelioration and prevention of various skin diseases, chapped skin, scaly skin, etc., and is highly safe.

EP 0 832 646 A1

## Description

FIELD OF THE INVENTION

This invention relates in general to external preparations for the amelioration and prevention of chapped skin, and more particularly to external preparations for the amelioration and prevention of chapped skin which has the amelioration and prevention effects on chapped skin symptoms due to various skin diseases such as contact dermatitis and psoriasis, as well as on chapped skin and scaly skin of healthy people, by blending in an extract of a seaweed of genus Ascophyllum as an effective ingredient.

BACKGROUND OF THE INVENTION

Conventionally, various remedies, external preparations and cosmetics have been known to have amelioration and prevention effects on skin diseases and chapped skin. These conventional drugs, cosmetics and such have been used because the effective ingredients with anti inflammatory actions or moisture preserving effects such as amino acids, polysaccharides, lipids and extracts had a superior ability to prevent inflammation of the skin and/or loss of moisture in corneum. However, none of these necessarily had enough amelioration and prevention effects on chapped skin and the development of even superior active agents has been desired.

Recently, it has become clearer that proteases (proteolytic enzymes) are involved in the development of various skin diseases. For example, in the case of psoriasis, which is a representative inflammatory dyskeratosis, a high plasminogen activator (PA) activity has been observed on the affected epidermis. The PA is a factor which specifically acts on plasminogen to convert it to active plasmin. A typical example of this factor is a protease such as serine protease. Examples of the relationship between this PA activity and skin diseases include a report of a strong PA activity in the epidermis with psoriasis, particularly in parakeratosis sites (Haustein, "Arch. Klin. Exp. Dermatol.": 234, 1969) and a report of extraction of the PA from psoriasis scales using a high concentration salt solution (Fraki, Hopsu-Havu, "Arch. Dermatol. Res."; 256, 1976). Also, an in vitro experimental system was used to reveal the fact that, in the case of pemphigus vulgaris, the PA is synthesized in large amounts in epidermal cells and converts plasminogen which exists outside of the cells into plasmin, which in turn digests the intercellular connective substance to cause intercellular accumulation of the tissue fluid, resulting in intraepidermal vesicles (Morioka S. et al., "J. Invest. Dermatol."; 76, 1981).

Also, it is believed that proteases play important roles in the normal cornification process of epidermis such as in the formation of the corneum (Ogawa H., Yoshiike T., "Int. J. Dermatol."; 23, 1984) and attempts have been made recently to use protease inhibitors as remedies for skin diseases or for skin amelioration.

Based on the aforementioned observations of the situation, the inventors carried out earnest investigations and came to believe that protease activity inhibitors were effective in causing amelioration in various skin diseases, chapped skin, scaly skin, etc., and thus they checked a wide variety of substances for their protease inhibition activity and discovered that the extract of a seaweed of genus Ascophyllum had a superior inhibition activity against proteases, particularly the trypsin-type serine proteases. Based on this finding, the present invention was completed.

DISCLOSURE OF THE INVENTION

The present invention provides external preparations for the amelioration and prevention of chapped skin which contains as an effective ingredient an extract of a seaweed of genus Ascophyllum.

Use of the extract of seaweed of genus Ascophyllum has already been reported as a antiblackening agent (Japanese unexamined patent publication Tokkai Hei 4-135472), an antioxidant (Tokkai Hei 4-239593), an anti-odor agent (Tokkai Hei 5-3907) and a freshness retainer for cut flowers (Tokkai Hei 5-194101). However, as far as the inventors know, there has not been any report regarding the protease inhibiting action and the application of it as a protease inhibitor or a chapped skin amelioration agent has not been known at all.

THE BEST MODES OF THE EMBODIMENTS

The present invention is described in detail below.

The seaweed of genus Ascophyllum which is used in the present invention is a seaweed of genus Ascophyllum fucales fucaceae phaeophyceas harvested in northern Europe, Canada, etc. Of these, Ascophyllum nodosum is more preferably used in the present invention.

The extract of seaweed of genus Ascophyllum can be obtained with a conventional method. For example, it can be obtained by immersion or heated refluxing of seaweed of genus Ascophyllum with an extraction solvent, followed by filtration and concentration. For the extraction solvent, any solvent normally used for extraction can be used. For example, alcohols including methanol, ethanol, propylene glycol, 1, 3-butylene glycol and glycerine, organic solvents including hydrated alcohol, acetone and ethyl acetate can be used individually or in combination. Also, the extract can be treated by means of purification with chromatography or distribution before use.

In this invention, the blend ratio of the Ascophyllum extract should preferably be 0.005-20.0 wt% (dry weight), more preferably 0.05-10.0 wt%, of the total external preparation. If it is less than 0.005 wt%, then there will not be a sufficient ameliorating and preventing chapped skin effect. On the other hand, if it is more than 20 wt%, then pharmaceutical preparation becomes difficult. Also, blending more than 10.0 wt% does not improve the effects significantly.

The external preparations for the amelioration and prevention of chapped skin of the present invention can be used as a protease inhibitor. "Protease" is a general term for enzymes which catalyze the hydrolysis of peptide bonds and they are classified into peptidases and proteinases. The former is an enzyme which severs peptide bonds in a peptide chain from outside, i.e. at the amino end or at the carboxyl end, and the latter is an enzyme which severs a specific bond inside a peptide chain. The latter, proteinases, are further classified into the serine type, cysteine type, aspartic acid type and metal type. For each of them there are specific inhibitors. The protease inhibitor of the present invention characteristically exhibits the inhibition activity against serine proteases in particular.

In addition to the essential ingredients described above, the external preparations for the amelioration and prevention of chapped skin of the present invention can contain, as necessary, those ingredients such as are normally used in cosmetics, drugs, etc. in the form of an external preparation, including water -based ingredients, oil-based ingredients, powder ingredients, alcohols, humectants, thickeners, ultraviolet light absorbents, whitening agents, preservatives, antioxidants, surfactants, perfumes, colorings and various skin nutrients.

In addition, sequestering agents including disodium edetate, trisodium edetate, sodium citrate, sodium metaphosphate, sodium metaphosphate and gluconic acid; drugs including caffeine, tannin, verapamil, Glycyrrhiza extract, glabridin, a hot water extract of quince fruit, various crude drugs, tocopherol acetate, glycyrrhizic acid, tranexamic acid and its derivatives or its salts; vitamin C, magnesium ascorbate phosphate, ascorbate glucoside, arbutin and kojic acid, and sugars including glucose, fructose and trehalose can also be added.

The external preparations for the amelioration and prevention of chapped skin of the present invention can be in any form which is usually used for an external preparation, including an ointment, cream, emulsion, lotion, facial packs or bath essences.

## EXAMPLES

The present invention is described in detail below by referring to examples. The technical scope of the present invention is not limited to these examples. All the blend ratios are in weight percent units.

Before explaining the examples, the testing methods and the evaluation criteria of the protease inhibition effect and the chapped skin ameliorating effect of the Ascophyllum extract of the present invention are described.

## I. Evaluation of the protease inhibition activity

The protease inhibition activity was evaluated using two representative serine proteases, plasmin and trypsin.

## (1) Preparation of the samples

50 g of dry powder of Ascophyllum nodusum was stirred in twice that amount of methanol at room temperature for one week. The obtained extract solution was filtered and the filtrate was concentrated to obtain 3.0 g of the methanol extract. This solid substance was then dissolved in dimethylsulfoxide (DMSO) to prepare a 3% solution. The following experiments were conducted using this.

## (2) Measurement of the plasmin inhibition activity

The inhibition ratio (%) was obtained by means of the fibrin flat plate method. 6 ml of Veronal buffer (25 mM sodium barbiturate solution containing 0.125 M NaOH, pH 7.4) containing 0.1% fibrinogen (plasminogen removed) was poured into a Petri dish, to which 0.2 ml of 1.0 M $CaCl_2$ and 0.1 ml of 25 U/ml thrombin were added and mixed gently. The mixture was then let stand for one hour. 20 microliters of a mixture of 5 U/ml plasmin and the specimen with a ratio of 29 : 1, after being kept at 37°C for 10 minutes, was added to the flat plate which was formed by fibrinogen's conversion to fibrin. The Petri dish was let stand for 18 hours at 37°C. For the control, the same operation was carried out using DMSO instead of the specimen. After the operations, the area of the dissolution circle formed by dissolution of fibrin was measured and the plasmin inhibition ratio was determined using the following equation 1:

The inhibition ratio (%) = 1 - (Area of the dissolution
circle for the specimen/Area of the dissolution circle for the control)

The results are shown in Table 1.

(3) Measurement of the trypsin inhibition activity

The inhibition ratio was determined by using casein as the substrate. 40 micrograms of trypsin were dissolved in 2 ml of a phosphate buffer. 0.9 ml of 0.1 M phosphate buffer (pH 7.4) containing 6% casein and 0.1 ml of the specimen were added to this, and the temperature was maintained at 37°C for 10 minutes. After this, 3 ml of 5% trichloroacetic acid was added and the mixture was let stand at room temperature for one hour. After centrifugation at 3,500 rpm for 15 minutes, the light absorption at 280 nanometers of the supernatant was measured. This operation was named 'Test (T)'. The operation in which the timing of the trypsin addition was moved to after the trichloroacetic acid addition was named 'Control (C)'. The operation in which DMSO was added instead of the specimen was named 'Standard (S)'. The operation similar to Standard except for the fact that the timing of the trypsin addition was moved to after the trichloroacetic acid addition was named Blank (B). The trypsin inhibition ratio was determined by the following equation 2:

$$\text{Inhibition ratio (\%)} = 1 - (T - C/S - B)$$

The results are shown in Table 1.

As a reference, extracts of Kunyit (Curcuma domestica) of the Zingiberaceae family and mugwort, whose application for chapped skin are already known, were tested in the same manner as described above. These results are also shown in Table 1.

Table 1

| Sample | | Inhibition ratio (%) | |
|---|---|---|---|
| | | Plasmin | Trypsin |
| Ascophyllum | 0.1% | 65.8 | 32.4 |
| | 0.01% | 34.8 | 11.7 |
| Kunyit | 0.1% | 3.0 | 0 |
| | 0.01% | 0 | 0 |
| Mugwort | 0.1% | 18.6 | 0 |
| | 0.01% | 5.8 | 0 |

The results shown in Table 1 clearly indicate that the Ascophyllum extract has a much superior serine protease inhibition activity than the extracts of Kuynit and mugwort.

II. Chapped skin amelioration testing

(1) Testing in actual use

The effects of the external preparation when it is applied on the endermis were evaluated in terms of chapped skin amelioration, sycosis amelioration and stimulation of the skin.

As shown in Table 2, two methanol extracts of Ascophyllum nodosum with different concentrations were blended in the samples of the present invention. For the comparative samples, methanol extracts of Kunyit (C. domestica) of the ginger family (Zingiberaceae), application of which for chapped skin was already known, and Lempuyang (Zingiber aromaticum Mal.) from the same family were blended in.

(Chapped skin amelioration ratio)

Testing was conducted on the faces of a panel of 50 women with chapped skin problems. Using lotions with the compositions (wt%) shown in Table 2, the sample was applied on either the left or right cheek and the control was applied on the other cheek twice a day for two weeks. After this, the conditions of the skin were evaluated by visual observation. The judgment criteria and the evaluation ratings were as follows.

(Judgment criteria of the chapped skin ameliorating effects)

Very effective: The symptoms disappeared.
Effective: The symptoms were alleviated.
Somewhat effective: The symptom were somewhat alleviated.
Not effective: No change in the symptoms.

[Evaluation of the chapped skin amelioration effects]

◎ : The ratio of the subjects who indicated 'very effective' or 'effective' (effective ratio) is 80 % or more.
○ : The ratio of the subjects who indicated 'very effective' or 'effective' (effective ratio) is 50 % or more and less than 80%.
△ : The ratio of the subjects who indicated 'very effective' or 'effective' (effective ratio) is 30 % or more and less than 50%
X : The ratio of the subjects who indicated 'very effective' or 'effective' (effective ratio) is less than 30%.

(Sycosis amelioration ratio)

A panel of 30 men with a sycosis problem caused by razors was used as subjects. Right after shaving, lotions with the compositions shown in Table 2 were applied and the effects against sycosis were judged. The judgment criteria and the evaluation ratings were as follows.

(Judgment criteria of the sycosis amelioration effects)

Very effective: The sycosis disappeared.
Effective: The sycosis was alleviated.
Somewhat effective: The sycosis was somewhat alleviated.
Not effective: No change in the sycosis.

[Evaluation of the chapped skin amelioration effects]

◎ : The ratio of the subjects who indicated 'very effective' or 'effective' (effective ratio) is 80 % or more.
○ : The ratio of the subjects who indicated 'very effective' or 'effective' (effective ratio) is 50 % or more and less than 80%.
△ : The ratio of the subjects who indicated 'very effective' or 'effective' (effective ratio) is 30 % or more and less than 50%.
X : The ratio of the subjects who indicated 'very effective' or 'effective' (effective ratio) is less than 30%.

(Skin stimulation characteristics)

When the aforementioned chapped skin amelioration ratio and the sycosis amelioration ratio were judged, the skin stimulation characteristics of the samples of the present invention and the comparative examples were also judged and evaluated. Evaluation criteria are shown below.

[Evaluation of skin stimulation]

◎ : The ratio of the subjects who reported stinging of the skin was 0%.
○ : The ratio of the subjects who reported stinging of the skin was less than 5%.
△ : The ratio of the subjects who reported stinging of the skin was less than 10%.
X : The ratio of the subjects who reported stinging of the skin was 10% or more.

The results are shown in Table 2.

Table 2

| Sample | | Samples of the present invention | | Comparative samples | |
|---|---|---|---|---|---|
| | | 1 | 2 | 1 | 2 |
| Methanol extract of Ascophyllum | | 1.0 | 0.5 | - | - |
| Methanol extract of Kunyit Methanol extract of | | - | - | 1.0 | - |
| Lempuyang | | - | - | - | 1.0 |
| Glycerine | | 1.0 | 1.0 | 1.0 | 1.0 |
| 1,3-butylene glycol | | 4.0 | 4.0 | 4.0 | 4.0 |
| Ethanol | | 7.0 | 7.0 | 7.0 | 7.0 |
| Polyoxyethylene (20-mole) oleyl alcohol | | 0.5 | 0.5 | 0.5 | 0.5 |
| Purified water | | Balance | Balance | Balance | Balance |
| Evaluation | Chapped skin improvement effect | ○ | ○ | △ | △ |
| | Sycosis improvement effect | ◎ | ○ | △ | △ |
| | Skin stimulation effect | ◎ | ◎ | ○ | △ |

As clearly shown in Table 2, compared with the comparative samples, the samples of the present invention which contained the Ascophyllum extract exhibited superior amelioration effects against chapped skin and sycosis, and no skin stimulation was reported.

(2) Testing in actual use with the replica method

Lotions of samples 1 and 2 of the present invention and comparative samples 1 and 2 were used on a human body panel for testing of the chapped skin amelioration effect. The skin surface conditions of the (faces of) healthy women were observed microscopically (17× magnification) by taking a replica of the skin with the replica method using myristin resin. The judgment criteria shown below were based on the conditions of dermatoglyphics and peeling of the corneum. 20 women who were given skin evaluation ratings of 1 and 2 (chapped skin panel) were chosen to apply the lotions of the samples of the present invention 1 and 2 and the comparative samples 1 and 2 on left and right halves of their faces once a day for two weeks. After two weeks, the skin conditions were again observed by means of the aforementioned replica method and evaluation was made according to the following judgment criteria. The results are shown in Table 3.

〈Judgment criteria〉

1 : The sulcus cutis and crista cut is disappeared and roll-up of the corneum over a wide area was observed.
2 : The sulcus cutis and crista cut is were unclear and roll-up of the corneum was observed.
3 : The sulcus cutis and crista cut is could be seen but were flat.
4 : The sulcus cutis and crista cutis were clear.
5 : The sulcus cutis and crista cutis were clear and orderly.

Table 3

| Replica evaluation | Samples of the present invention (number of subjects) | | Comparative samples (number of subjects) | |
|---|---|---|---|---|
| | 1 | 2 | 1 | 2 |
| 1 | 0 | 0 | 1 | 1 |
| 2 | 0 | 2 | 3 | 2 |
| 3 | 7 | 10 | 14 | 11 |
| 4 | 11 | 8 | 2 | 6 |
| 5 | 2 | 0 | 0 | 0 |

As clearly shown in Table 3, the lotions of the present invention exhibited remarkable chapped skin amelioration effects compared with the lotions of the comparative samples.

Example 1 : Cream

| (Recipe) | Blend ratio |
|---|---|
| (1) Stearic acid | 5.0 |
| (2) Stearyl alcohol | 4.0 |
| (3) Isopropyl myristate | 18.0 |
| (4) Glycerine monostearic ester | 3.0 |
| (5) Propylene glycol | 10.0 |
| (6) Ascophyllum methanol extract | 0.01 |
| (7) Caustic potash | 0.2 |
| (8) Sodium hydrogen sulfite | 0.01 |
| (9) Preservative | Appropriate amount |
| (10) Perfume | Appropriate amount |
| (11) Ion exchange water | Balance |

(Preparation method)

The ingredients (5)-(7) were added to the ingredient (11) and the mixture was heated to and kept at 70°C (water phase). The ingredients (1)-(4) and (8)-(10) were mixed, then heat-melted and the temperature was kept at 70°C (oil phase). The oil phase was gradually added to the water phase, and after all of it had been added, the temperature was kept at that temperature to allow the mixture to react. Finally, the mixture was homogeneously emulsified by a homomixer and cooled to 30°C while being thoroughly stirred.

Example 2 : Cream

| (Recipe) | Blend ratio |
|---|---|
| (1) Stearic acid | 2.0 |
| (2) Stearyl alcohol | 7.0 |
| (3) Hydrated lanolin | 2.0 |
| (4) Squalene | 5.0 |
| (5) 2-octyldodecyl alcohol | 6.0 |
| (6) Polyoxyethylene (25-mole) cetyl alcohol ether | 3.0 |
| (7) Glycerine monostearic ester | 2.0 |
| (8) Propylene glycol | 5.0 |
| (9) Ascophyllum ethanol extract | 0.05 |
| (10) Sodium hydrogen sulfite | 0.03 |
| (11) Ethyl paraben | 0.3 |
| (12) Perfum | Appropriate amount |
| (13) Ion exchange water | Balance |

(Preparation method)

The ingredient (8) was added to the ingredient (14) and the mixture was heated to and kept at 70 °C (water phase). The ingredients (1)-(7) and (9)-(13) were mixed, then heat-melted and the temperature was kept at 70°C (oil phase). The oil phase was added to the water phase, and after pre -emulsification, the mixture was homogeneously emulsified by a homomixer and cooled to 30°C while being thoroughly stirred.

Example 3 : Cream

| (Recipe) | Blend ratio |
|---|---|
| (1) Solid paraffin | 5.0 |
| (2) Bees wax | 10.0 |
| (3) Vaseline | 15.0 |
| (4) Liquid paraffin | 41.0 |
| (5) Glycerine monostearic ester | 2.0 |
| (6) Polyoxyethylene (20-mole) sorbitan monolauric ester | 2.0 |
| (7) Soap powder | 0.1 |
| (8) Borax | 0.2 |
| (9) Ascophyllum acetone extract | 0.05 |
| (10) Sodium hydrogen sulfite | 0.03 |
| (11) Ethyl paraben | 0.3 |
| (12) Perfume | Appropriate amount |
| (13) Ion exchange water | Balance |

(Preparation method)

The ingredients (7) and (8) were added to the ingredient (13) and the mixture was heated to and kept at 70°C (water phase). The ingredients (1)-(6) and (9)-(12) were mixed, then heat-melted and the temperature was kept at 70°C (oil phase). The oil phase was gradually added to the water phase to allow the reaction to occur. After this, the mixture was homogeneously emulsified by a homomixer and then cooled to 30°C while being thoroughly stirred.

Example 4 : Emulsion

| (Recipe) | Blend ratio |
|---|---|
| (1) Stearic acid | 2.5 |
| (2) Cetyl alcohol | 1.5 |
| (3) Vaseline | 5.0 |
| (4) Liquid paraffin | 10.0 |
| (5) Polyoxyethylene (10-mole) monooleic ester | 2.0 |
| (6) Polyethylene glycol (1500) | 3.0 |
| (7) Triethanol amine | 1.0 |
| (8) Carboxyvinyl polymer (Product name: Carbopol 941 from B. F. Goodrich Chemical company) | 0.05 |
| (9) Ascophyllum ethyl acetate extract | 0.01 |
| (10) Sodium hydrogen sulfite | 0.01 |
| (11) Ethyl paraben | 0.3 |
| (12) Perfume | Appropriate amount |
| (13) Ion exchange water | Balance |

(Preparation method)

The ingredient (8) was dissolved in a small amount of the ingredient (13) (A phase). The ingredients (6) and (7) were added to and heat-dissolved in the rest of the ingredient (13), and the temperature was kept at 70°C (water phase). The ingredients (1)-(5) and (9)-(12) were mixed, then heat-melted and the temperature was kept at 70°C (oil phase). The oil phase was added to the water phase, and, after pre-emulsification, the A phase was added and the mixture was homogeneously emulsified by a homomixer and, after the emulsification, cooled to 30°C while being thoroughly stirred.

Example 5 : Emulsion

| (Recipe) | Blend ratio |
|---|---|
| (1) Microcrystalline wax | 1.0 |
| (2) Bees wax | 2.0 |
| (3) Lanolin | 20.0 |
| (4) Liquid paraffin | 10.0 |
| (5) Squalene | 5.0 |
| (6) Sorbitan sesquioleic ester | 4.0 |
| (7) Polyoxyethylene (20-mole) sorbitan monooleic ester | 1.0 |
| (8) Propylene glycol | 7.0 |
| (9) Ascophyllum butanol extract | 5.0 |
| (10) Tranexamic acid | 1.0 |
| (11) Sodium hydrogen sulfite | 0.01 |
| (12) Ethyl paraben | 0.3 |
| (13) Perfume | Appropriate amount |
| (14) Ion exchange water | Balance |

(Preparation method)

The ingredient (8) was added to the ingredient (14) and the mixture was heated to and kept at 70 °C (water phase). The ingredients (1)-(7) and (9)-(13) were mixed, then heat-melted and the temperature was kept at 70°C (oil phase). The oil phase was gradually added to the water phase while stirring was conducted, and the mixture was homogeneously emulsified by a homomixer and then cooled to 30°C while being thoroughly stirred.

Example 6 : Jelly

| (Recipe) | Blend ratio |
|---|---|
| (1) 95% ethyl alcohol | 10.0 |
| (2) Dipropylene glycol | 15.0 |
| (3) Polyoxyethylene (50-mole) oleyl alcohol ether | 2.0 |
| (4) Carboxyvinyl polymer (Product name: Carbopol 940 from B. F. Goodrich Chemical company) | 1.0 |
| (5) Caustic soda | 0.15 |
| (6) L-arginine | 0.1 |
| (7) Ascophyllum 50% methanol aqueous solution extract | 7.0 |
| (8) Sodium hydrogen sulfite | 0.01 |
| (9) Ethyl paraben | 0.3 |
| (10) Perfume | Appropriate amount |
| (11) Ion exchange water | Balance |

(Preparation method)

The ingredient (4) was homogeneously dissolved in the ingredient (11). The ingredients (7) and (3) were dissolved in the ingredient (1) and this mixture was added to the water phase. The ingredients (2) and (8)-(10) were then added, and the mixture was neutralized and thickened with the ingredients (5) and (6).

Example 7 : Jelly

| (Recipe) | Blend ratio |
|---|---|
| (A phase) | |
| Ethyl alcohol (95%) | 10.0 |
| Polyoxyethylene (20-mole) octyl dodecanol | 1.0 |
| Pantothenyl ethyl ether | 0.1 |
| Ascophyllum methanol extract | 1.5 |
| Methyl paraben | 0.15 |
| (B phase) | |
| Potassium hydroxide | 0.1 |
| (C phase) | |
| Glycerine | 5.0 |
| Dipropylene glycol | 10.0 |
| Sodium hydrogen sulfite | 0.03 |
| Carboxyvinyl polymer (Product name: Carbopol 940 from B. F. Goodrich Chemical company) | 0.2 |
| Purified water | Balance |

(Preparation method)

The A phase and the C phase were independently dissolved homogeneously, and then the A phase was added to the C phase and solubilized. The B phase was then added, and finally containers were filled.

Example 8 : Facial pack

| (Recipe) | Blend ratio |
|---|---|
| (A phase) | |
| Dipropylene glycol | 5.0 |
| Polyoxyethylene (60-mole) hardened castor oil | 5.0 |
| (B phase) | |
| Ascophyllum acetone extract | 0.01 |
| Olive oil | 5.0 |
| Tocopherol acetate | 0.2 |
| Ethyl paraben | 0.2 |
| Perfume | 0.2 |
| (C phase) | |
| Sodium hydrogen sulfite | 0.03 |
| Polyvinyl alcohol (Degree of saponification 90, degree of polymerization 2,000) | 13.0 |
| Ethanol | 7.0 |
| Purified water | Balance |

(Preparation method)

The A, B and C phases were independently dissolved homogeneously, and then the B phase was added to the A phase and solubilized. The C phase was then added and finally containers were filled.

Example 9 : Solid foundation

| (Recipe) | Blend ratio |
|---|---|
| (1) Talc | 43.1 |
| (2) Kaolin | 15.0 |
| (3) Sericite | 10.0 |
| (4) Zinc flower | 7.0 |
| (5) Titanium dioxide | 3.8 |
| (6) Yellow iron oxide | 2.9 |
| (7) Black iron oxide | 0.2 |
| (8) Squalene | 8.0 |
| (9) Isostearic acid | 4.0 |
| (10) POE sorbitan monooleate | 3.0 |
| (11) Isocetyl octate | 2.0 |
| (12) Ascophyllum ethanol extract | 1.0 |
| (13) Preservative | Appropriate amount |
| (14) Perfume | Appropriate amount |

(Preparation method)

The powder ingredients, i.e. from the ingredients (1) to (7), were thoroughly mixed by a blender, and the oil-based ingredients, i.e. (8)-(11), and (12), (13) and (14) were added to this. After a thorough kneading, the product was filled into a container and molded.

Example 10 : Emulsified foundation

| (Recipe) | Blend ratio |
|---|---|
| (Powder portion) | |
| Titanium dioxide | 10.3 |
| Sericite | 5.4 |
| Kaolin | 3.0 |
| Yellow iron oxide | 0.8 |
| Red iron oxide | 0.3 |
| Black iron oxide | 0.2 |
| (Oil phase) | |
| Decamethylpentasiloxane | 11.5 |
| Liquid paraffin | 4.5 |
| Polyoxyethylene modified dimethylpolysiloxane | 4.0 |
| (Water Phase) | |
| Purified water | 50.0 |
| 1,3-butylene glycol | 4.5 |
| Ascophyllum methanol extract | 1.5 |
| Sorbitan sesquioleic ester | 3.0 |
| Preservative | Appropriate amount |
| Perfume | Appropriate amount |

(Preparation method)

After heating and stirring the water phase, the powder portion, thoroughly mixed and crushed, was added to it and the mixture was treated with a homomixer. The heat-mixed oil phase was then added to this mixture and the resulting mixture was treated with a homomixer. Finally, the perfume was added while the mixture was stirred and the temperature was lowered to room temperature.

INDUSTRIAL APPLICABILITY OF THE INVENTION

As described thus far, the present invention provides an external preparation which has a superior chapped skin amelioration action and superior effects on amelioration and prevention of various skin diseases which result in chapped skin and healthy people's chapped skin and scaly skin, exhibits a lower level of skin stimulation and is highly safe.

**Claims**

1. An external preparation for the amelioration and prevention of chapped skin which contains as an effective ingredient an extract from a seaweed of genus Ascophyllum.

2. An external preparation for the amelioration and prevention of chapped skin of claim 1 wherein said seaweed of genus Ascophyllum is Ascophyllum nodosum.

3. An external preparation for the amelioration and prevention of chapped skin of claim 1 or claim 2 wherein the blend ratio of said extract is 0.005 -20.0 wt% by dry weight of the total external preparation.

4. An external preparation for the amelioration and prevention of chapped skin of claim 1, 2 or 3 wherein said external preparation is a cosmetic.

5. A method of ameliorating and preventing chapped skin which uses the external preparations for the amelioration and prevention of chapped skin of claim 1, 2, 3 or 4.

## INTERNATIONAL SEARCH REPORT

| International application No. |
| --- |
| PCT/JP97/01115 |

### A. CLASSIFICATION OF SUBJECT MATTER
Int. Cl$^6$  A61K7/48, 7/00

According to International Patent Classification (IPC) or to both national classification and IPC

### B. FIELDS SEARCHED

Minimum documentation searched (classification system followed by classification symbols)

Int. Cl$^6$  A61K7/48, 7/00

Documentation searched other than minimum documentation to the extent that such documents are included in the fields searched

| | |
| --- | --- |
| Jitsuyo Shinan Koho | 1926 – 1997 |
| Kokai Jitsuyo Shinan Koho | 1971 – 1997 |
| Toroku Jitsuyo Shinan Koho | 1994 – 1997 |

Electronic data base consulted during the international search (name of data base and, where practicable, search terms used)

CAS ONLINE

### C. DOCUMENTS CONSIDERED TO BE RELEVANT

| Category* | Citation of document, with indication, where appropriate, of the relevant passages | Relevant to claim No. |
| --- | --- | --- |
| Y | Chemical Abstracts, Vol. 79, abstract No.: 129066, 1973 (Refer to Maroc Med., 53(567), 233-8 issued 1973) | 1 – 5 |
| Y | JP, 7-274977, A (Shikoku Research Instiute Inc.), October 24, 1995 (24. 10. 95)(Family: none) | 1 – 5 |
| Y | JP, 8-73314, A (Ichimaru Falcos K.K.), March 9, 1996 (09. 03. 96)(Family: none) | 1 – 5 |

☐ Further documents are listed in the continuation of Box C.　　☐ See patent family annex.

| | |
| --- | --- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| June 24, 1997 (24. 06. 97) | July 8, 1997 (08. 07. 97) |

| Name and mailing address of the ISA/ | Authorized officer |
| --- | --- |
| Japanese Patent Office | |
| Facsimile No. | Telephone No. |

Form PCT/ISA/210 (second sheet) (July 1992)